# EUROPÄISCHE PATENTANMELDUNG

(11) **EP 4 552 638 A2**
(43) Veröffentlichungstag der Anmeldung: **14.05.2025**
(21) Anmeldenummer: 25160774.3
(22) Anmeldetag: 16.01.2023
(51) Int. Cl.: A61K 31/341

(54) **TOFA-ANALOGA, ZUBEREITUNGEN ZUR SEBUMREDUKTION MIT EINEM GEHALT AN SOLCHEN ANALOGA UND DIE KOSMETISCHE UND/ODER THERAPEUTISCHE VERWENDUNG SOLCHER ANALOGA ALS WIRKSAMES PRINZIP ZUR SEBUMREDUKTION ODER -VERHINDERUNG**

(30) Priorität: 08.02.2022 DE 102022201276
(62) Teilanmeldung aus: 23701009.5
(71) Anmelder: Beiersdorf AG, 22529 Hamburg (DE)
(72) Erfinder: Seidel, Judith, 13507 Berlin (DE); Reuter, Jörn Hendrik, 24558 Henstedt Ulzburg (DE); Kamal, Ahmed, 22043 Hamburg (DE); Simmering, Annika, 20249 Hamburg (DE); Mielke, Heiko, 21629 Neu Wulmstorf (DE)
(74) Vertreter: Beiersdorf AG

(57) **Zusammenfassung**

Verbindungen der allgemeinen Formel

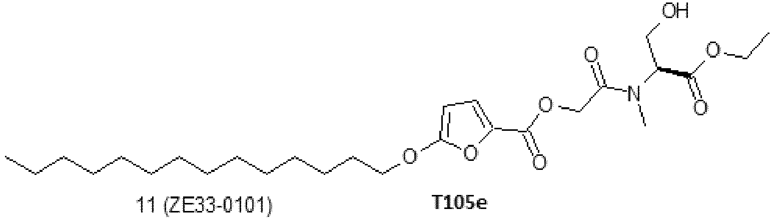

## Beschreibung

Die vorliegende Erfindung betrifft neue Substanzen und deren Verwendungen als Wirkstoffe und Zubereitungen, solche Wirkstoffe enthaltend, welche gegen fettige und/oder unreine Haut, sowie milden Formen der Akne und/oder bei fettigem Haar wirksam sind.

Seborrhö ist eine gesteigerte Funktion der Talgdrüsen durch Veranlagung. Sowohl Kopfhaut als auch Gesichtshaut erscheinen fettig. Die Zusammensetzung des seborrhöischen Sebums ist gegenüber dem normalen Sebum verändert. man unterscheidet 3 Entwicklungsstufen der Seborrhö:
1. Einfache Seborrhö: Leichte Fälle, fettig nach 8 Tagen.
2. Ölige Seborrhö: Bereits nach 2-3 Tagen fettig.
3. Irreversible Form: Nicht mehr umkehrbar. Die Seborrhö, bei der das Haar bereits nach einem Tag wie in Fett gebadet aussieht.

Die übermäßige Absonderung der Talgdrüsen kann unter anderem durch androgenetische Störungen (männliche Geschlechtshormonstörung) ausgelöst werden und hat einen ästhetischen Nachteil auf das Gesamtbild der Haare. Diese Störung kann auch die Ursache für auftretenden Haarausfall sein. Vorläufer ist jeweils der seborrhöische Zustand der Kopfhaut. Vegetative Störungen sowie unsachgemäße Pflege können das Hautbild und auch den Haarzustand noch verschlechtern. Auch bei Seborrhö kann das Haar selbst durch Störungen im Keratinaufbau trocken sein. Trockenes, angegriffenes Haar wird häufig hervorgerufen durch äußere Beanspruchung wie z. B. Sonne oder chemischen Behandlungen. Zu heißes Föhnen oder nicht richtige Pflege von angegriffenem Haar können zu Schädigungen führen.

Die Ursachen für fettiges Haar liegen im Körper des Menschen und sind hormonell bedingt. Jedes Haar besitzt eine eigene Talgdrüse, die Fett (auch Sebum oder Talg genannt) produziert. Die Sebum-Produktion wird hormonell gesteuert, und es kann je nach Hormonempfindlichkeit der Talgdrüse zu einer Über- oder Unterproduktion kommen. Der Talg selbst hat die Funktion die Kopfhaut geschmeidig zu halten. Er gelangt aus der Talgdrüse auf die Kopfhaut und erst später den Haaransatz. Dort wird er normalerweise vom Haarschaft aufgenommen und bleibt unsichtbar. Bei einer Talgüberproduktion ist der Haarschaft nicht mehr in der Lage diesen aufzunehmen. Er wird als Fettfilm am Haar sichtbar. Die Folge ist strähniges, fettig glänzendes Haar.

Dadurch dass die Talgdrüsenproduktion vom Hormonhaushalt abhängig ist, lässt sich das Problem fettigen Haares nicht grundsätzlich lösen, denn die Talgdrüsen produzieren stetig Fett. Konsequente Pflege und hochwertige Pflegeserien sind immer noch die beste Methode zur Bekämpfung fettigen Haares.

Leider hat fettiges Haar sehr lästige Auswirkungen. Die Haare werden schon kurze Zeit nach dem Waschen wieder strähnig und die Frisur hält nicht.

Entgegen landläufiger Meinung ist es nur ein Gerücht, dass durch zu häufiges Waschen das Haar noch schneller fettig wird. Milde Shampoos gegen fettiges Haar sorgen dafür, dass übermäßiges Fett entfernt wird. Haar und Kopfhaut werden mit ausreichend Feuchtigkeit versorgt und gleichen die Überproduktion der Talgdrüsen aus.

Fettiges Haar und Schuppenbildung gehören zu den häufigsten Haarproblemen. Diese Anomalien sind auf eine Störung der Talgdrüsentätigkeit zurückzuführen. Bei einer Überfunktion der Talgdrüsen sprechen wir von einer Seborrhö. Dabei sind zwei Formen zu unterscheiden: die ölige Form (Seborrhoea oleosa) und die trockene Form (Seborrhoea sicca).

### Die Seborrhoea oleosa:

Hier liegt eine Überfunktion der Talgdrüsen vor, bei der die Talgdrüsen zu viel und zu öligen Talg erzeugen. Die Haut zeigt deshalb einen fettigen Glanz, und die Haare sind schon 2 bis 3 Tage nach der Wäsche wieder bis in die Spitzen fettig und strähnig.

### Die Seborrhoea sicca:

Sie ist ebenfalls auf eine Überfunktion der Talgdrüsen zurückzuführen, aber der Hauttalg ist trockener, er hat eine festere Konsistenz. Mit den Schüppchen der Oberhaut bildet er große, leicht zerreibbare Talgschuppen. Die Kopfhaut zeigt einen wachsartigen Glanz, das Haar fettet nur am Ansatz nach, die Längen und besonders die Spitzen sind trocken und sogar spröde.

Zur Behandlung der Seborrhö gehört zunächst mal die regelmäßige und gründliche Kopfwäsche mit Spezialshampoos, die so oft durchgeführt werden kann, wie es nötig erscheint. Die Wäsche sollte mit einer Massage im Bindegewebe verbunden sein, weil dadurch dir Talgdrüsen stärker entleert werden, was die Nachfettung verzögert.

Bei der unreinen Haut sowie bei milden Formen der Akne sind neben einer verstärkten Sebumproduktion auch andere Einflüsse wie z. B. bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen. Da ein wesentlicher Faktor dabei die übermäßige Talgproduktion ist, kann in vielen Fällen bereits durch den Einsatz von sebumreduzierenden Wirkstoffen eine Verbesserung des Hautzustandes erreicht werden.

Aufgabe der vorliegenden Erfindung war es also, gegen fettige und/oder unreine Haut, milde Formen der Akne sowie gegen fettiges Haar, wirksame Zubereitungen zu finden.

Der Stand der Technik kennt bereits einige Versuche zur Lösung dieses Problems, die jedoch nicht alle Vorteile der hier vorgestellten Entwicklung kennen.

Die WO 2011/005660 offenbart Analoga von 5-(Tetradecyloxy)-2-furancarbonsäure (TOFA) und deren antiseborrhöische Wirkung.

Die Schrift WO 2018/022797 offenbart Analoga von 5-(Tetradecyloxy)-2-furancarbonsäure (TOFA) und deren Wirkung gegen Acne Vulgaris.

Die Schrift WO 2019/115405 offenbart Analoga von Pyrrolderivaten und deren antiseborrhöische Wirkung.

Es wurde aber überraschend gefunden, und darin liegt die Lösung der Aufgabe, dass neue Verbindungen gemäß Anspruch 1
sowie medizinische oder kosmetische topische Zubereitungen, enthaltend eine oder mehrere solcher Verbindungen in einer wirksamen Konzentration,
sowie die kosmetische Verwendung einer oder mehrerer solcher Verbindungen zur Verringerung der Sebumproduktion in der menschlichen Haut,
sowie die Verwendung von topischen Zubereitungen enthaltend einer oder mehrerer solcher Verbindungen zur Behandlung von fettiger und/oder unreiner Haut und/oder milden Formen der Akne und/oder fettigem Haar und/oder fettiger Kopfhaut und/oder der Seborrhö,

den Nachteilen des Standes der Technik abhelfen.

Die erfindungsgemäßen Verwendungen folgen einem gleichartigen Prinzip, da die Talgdrüsen auf der Kopfhaut und im Gesicht identisch aufgebaut sind und die Sebumproduktion nach demselben Mechanismus abläuft. So kann durch die Anwendung der sebumreduzierenden Wirkstoffe auf der Kopfhautregion die Sebumproduktion gesenkt und somit der kosmetisch unerwünschte Zustand der fettigen Haare verhindert werden.

Erfindungsgemäß sind solche Zubereitungen besonders vorteilhaft, die dadurch gekennzeichnet sind, dass eine oder mehrere Verbindungen der allgemeinen Formel gemäß Anspruch 1, vorteilhaft in einer Wasser- und/oder Ölphase, in Konzentrationen von 0,0001 - 40,00 Gew.-%, bevorzugt 0,005 - 20,00 Gew.-%, besonders bevorzugt 0,001 - 6,00 Gew.-%, jeweils bezogen auf das Gesamtgewicht der Zusammensetzung, vorliegen.

### Synthese von TOFA (3)

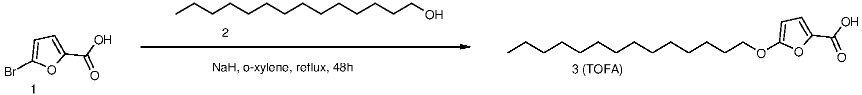

### Verbindung 3 (TOFA)

Zu einer Suspension von Alkohol (2) (25,3 g, 117,8 mM) in 400 ml o-Xylol wurde NaH (60 % in Mineralöl, 7,85 g, 196,3 mM) bei Umgebungstemperatur zugegeben und die Mischung wurde 2 Stunden lang unter Rückflusskühlung gerührt. Die Mischung wurde auf ca. 70ºC herunter gekühlt, und Säure (1) wurde als Feststoff in einer Portion (15,0 g, 78,5 mM) zugegeben. Die Mischung wurde 48 h unter Rückflusskühlung gerührt und auf Umgebungstemperatur abgekühlt. Die Mischung wurde mit wässrigem KHSO₄ gequencht, mit EtOAc extrahiert, die organische Schicht wurde zusätzlich mit H₂O gewaschen, mit Na₂SO₄ getrocknet und eingedampft. Der Rückstand wurde mit Hexan verrieben, was Verbindung (3) (7,1 g, 28 %) ergab. Die Synthese wurde mehrmals wiederholt, um die erforderliche Menge an TOFA (ca. 100 g) zu erhalten.

### ZE33-0101 (BDF: T105e)

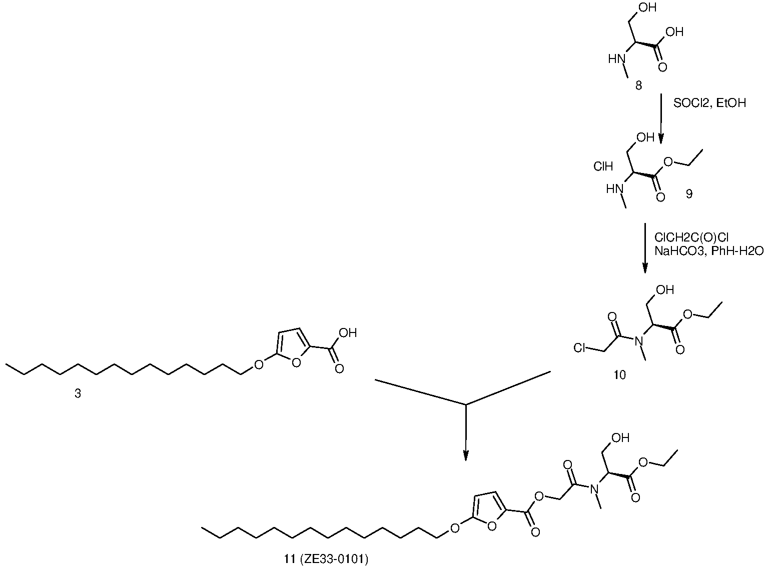

### Verbindung (9)

Zu einer Lösung von Verbindung (8) (2,0 g, 12,9 mM) in EtOH (20 ml) bei 0°C wurde tropfenweise SOCl₂; (7,6 g, 64,5 mM) zugegeben. Die resultierende Mischung wurde 12 h unter Rückflusskühlung erhitzt. Anschließend wurde die Mischung zum Abkühlen bei Raumtemperatur stehen gelassen und dann im Vakuum eingeengt. Nach Entfernung des Lösungsmittels wurde der Rückstand in wässriges NaHCO₃ gegossen. Die wässrige Lösung wurde mit DCM extrahiert, und der Extrakt wurde nacheinander mit wässrigem NaHCO₃, H₂O, Kochsalzlösung gewaschen und über Na₂SO₄ getrocknet. Aufkonzentration unter vermindertem Druck ergibt Verbindung (9) (2,2 g, 93%) als farbloses Pulver, das direkt für die nächste Reaktion verwendet wurde.

### Verbindung (10)

Verbindung (9) (2,2 g, 12,5 mM) wurde in 40 ml Wasser gelöst. Die Lösung wurde mittels eines Eisbades gekühlt und mit (2,6 g, 31,3 mM) NaHCO₃ in mehreren Schritten versetzt. Das Reaktionsgemisch wurde tropfenweise mit einer Lösung (1,3 g, 11,9 mM) von Chloracetylchlorid in 25 ml Toluol versetzt, dann 3 h bei 20-25°C kräftig gerührt. Nach einer Phasentrennung wurde die wässrige Phase mit Toluol extrahiert. Die vereinigten organischen Phasen wurden vom Lösungsmittel befreit, Verbindung (10) wurde als farbloses Öl (2,5 g, 90%) erhalten.

### Verbindung (11) (ZE33-0101) (BDF: T105e)

Zu einer Lösung von Verbindung (10) (10,0 g, 30,8 mM) in DMF (150 ml) wurde bei Raumtemperatur 4 (1,1 Äq., 7,6 g, 33,9 mM) und Triethylamin (2 Äq., 6,3 ml, 62,6 mM) zugegeben. Nach der Zugabe wurde das Gemisch 18 h bei 50 °C gerührt und dann wurde das Gemisch mit Ether (300 ml) verdünnt und mit Wasser (3 × 50 ml) gewaschen. Die zwei Schichten wurden getrennt und die wässrige Phase wurde mit Dichlormethan (2 × 50 ml) extrahiert. Die organischen Schichten wurden vereinigt, über wasserfreiem MgSO₄ getrocknet und unter reduziertem Druck eingedampft, um ein oranges Öl zu ergeben. Das resultierende Material wurde durch Silicagel-Säulenchromatographie (unter Elution mit 10-20% EtOAc/DCM) gereinigt, um Verbindung (11) (ZE33-0101) als weiße Kristalle (10,0 g, 63,4%) zu erhalten.

Die erfindungsgemäßen Verbindungen können vorteilhaft in kosmetische oder dermatologische Zubereitungen eingearbeitet werden.

Die erfindungsgemäßen Zubereitungen bzw. Verwendungen können in Form von mittels Pinsel oder Abstreifern, Roll-on-Vorrichtungen oder Zerstäubern auftragbaren flüssigen Zusammensetzungen, als Stifte und in Form von aus normalen Flaschen und Behältern auftragbaren Systemen, z. B. Crèmes, Gelen oder Lotionen vorliegen. So können sie z. B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Weiterhin können die erfindungsgemäßen Zubereitungen bzw. Verwendungen vorteilhaft in Form von Gesichtswässern, Tinkturen, Reinigungsformulierungen, Pads, Wattebäuschen oder Tüchern sowie in Form von Tonics oder Shampoos vorliegen.

Vorteilhaft wird der pH-Wert der erfindungsgemäßen Zubereitungen bzw. Verwendungen im schwach sauren bis neutralen Bereich eingestellt, bevorzugt von 3,0 - 7,0, besonders bevorzugt von 4,0 - 6,5.

Die erfindungsgemäßen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden; beispielsweise Konsistenzgeber, Konservierungsmittel, Stabilisatoren, Füllstoffe, Parfüme, Pigmente, die färbende Wirkung haben, Verdickungsmittel, Suspendiermittel, Puffergemische, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Wasser, Salze, antimikrobiell, proteolytische oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Die jeweils einzusetzenden Mengen an Trägerstoffen können in Abhängigkeit von der Art des jeweiligen Produktes vom Fachmann durch einfaches Ausprobieren leicht ermittelt werden.

Als übliche Trägerstoffe zur Herstellung der erfindungsgemäßen Zubereitungen bzw. Verwendungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden, sowie schleimbildende Stoffe und Verdickungsmittel, z. B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Emulgatoren zur Herstellung der erfindungsgemäßen Zubereitungen bzw. Verwendungen, welche vorteilhaft als flüssige oder feste Zubereitungen auf die gewünschten Hautbereiche aufgetragen werden sollen, und die in den Zubereitungen in geringer Menge, z.B. 1 bis 6 Gew.-%, bezogen auf die Gesamt-Zusammensetzung, verwendet werden können, haben sich nichtionogene Typen, wie Polyoxyethylen-Fettalkoholether, z. B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z. B. Sorbitanmonostearat und Polyoxyethylensorbitanmonostearat) und langkettige höhermolekulare wachsartige Polyglykolether als geeignet erwiesen. Daneben sind aber auch eine ganze Reihe von anderen Emulgatoren oder Emulgatormischungen geeignet, welche üblicherweise in kosmetischen Zubereitungen Verwendung finden. Dazu zählen beispielsweise, aber nicht beschränkend auf, Glycerylstearatcitrat, PEG-40 Stearat oder auch Polyglyceryl(3)-Methylglucosedistearat, Stearinsäure, Steareth-2 und Steareth-21.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Verbindungen der Ölphase können synthetischer, halbsynthetischer oder natürlicher Herkunft sein.

Auch beliebige Abmischungen solcher Öl- und Wachskomponenten sind vorteilhaft im Sinne der vorliegenden Erfindung einzusetzen.

Vorteilhaft beträgt der Gehalt an der Ölphase zwischen 1 und 50 Gew.-%, bezogen auf das Gesamtgewicht der Zubereitungen, bevorzugt 2 - 30 Gew.-%, insbesondere bevorzugt unter 10 Gew.-%.

Zur Anwendung werden die erfindungsgemäßen kosmetischen und/oder dermatologischen Formulierungen in der für Kosmetika und Dermatika üblichen Weise auf die Haut, Kopfhaut und/oder die Haare in ausreichender Menge aufgebracht.

### Lipidassay zur Testung der Wirksamkeit erfindungsgemäßer TOFA-Derivate

Für die Bestimmung des Lipid-reduzierenden Effekts von Wirkstoffen werden primäre Zelllinien von Talgdrüsen unterschiedlicher menschlicher Spender verwendet. Die Zellen werden für insgesamt 7 Tage kultiviert. Nachdem die Zellen an Tag 1 ausgesät werden, erfolgt die Wirkstoffbehandlung an Tag 3 und Tag 5. An Tag 7 werden die Zellen mit dem Farbstoff AdipoRed angefärbt. Dieser ermöglicht es die Lipidtröpfchen im Inneren der Zellen fluoreszent anzufärben und zu quantifizieren. Zur Kontrolle der Vitalität der Zellen wird außerdem der Farbstoff Fluoresceindiacetat (FDA) verwendet, der von lebenden Zellen umgesetzt wird. Das entstehende Fluoreszenzsignal kann ebenfalls quantifiziert werden. Der Effekt eines Wirkstoffes kann als Ratio des AdipoRed und FDA Fluoreszenzsignals relativ zu der Ratio des entsprechenden Lösungsmittels gemessen und dargestellt werden. Die getesteten Substanzen wurden in Dimethylsulfoxid (DMSO) gelöst, dementsprechend wurden alle Ergebnisse auf die mit DMSO behandelte Kontrolle normiert.

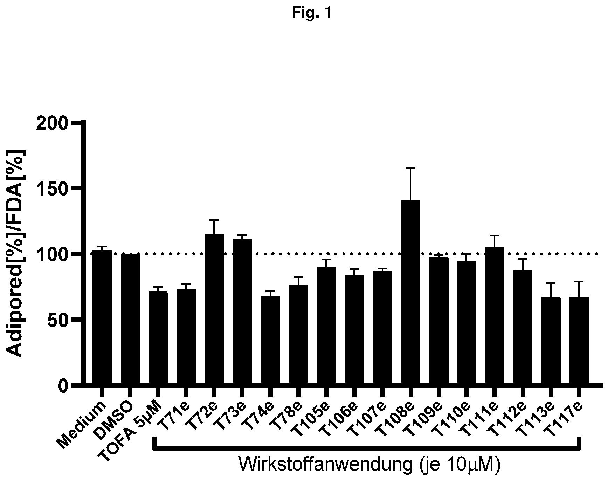

Fig. 1 zeigt die Ergebnisse des Assays.

Die folgenden Beispiele sollen die Erfindung erläutern, aber nicht einschränken. Die Zahlenangaben beziehen sich auf Gew.-%, sofern nichts anderes angegeben ist. Die Beispiele 2, 3, 4, 6 und 7 sind nicht Teil der Erfindung

| | Beispiel 1 | Beispiel 2 | Beispiel 3 | Beispiel 4 |
|---|---|---|---|---|
| Caprylic/Capric Triglyceride | | | | |
| Propylene Glycol + Aqua | | | 5,00 | 5,00 |
| Citric Acid | | 0,00 | 0,00 | 0,00 |
| Cetearyl Alcohol | 2,00 | | | |
| Aqua + Trisodium EDTA | 1,00 | | | |
| Phenoxyethanol + Aqua | 0,40 | | | |
| Dimethicone | 3,00 | | | |
| Glycerin | 6,00 | 4,00 | 4,00 | 10,00 |
| PEG-40 Hydrogenated Castor Oil | | | 1,00 | 5,00 |
| Butylene Glycol + Aqua | | 20,00 | 10,00 | 10,00 |
| Hydroxyethylcellulose + Disodium Phosphate + Sodium Phosphate | 0,15 | | | |
| Alcohol Denat. + Aqua | 8,00 | 50,00 | 40,00 | 40,00 |
| Xanthan Gum | 0,15 | | | |
| Acrylates/C10-30 Alkyl Acrylate Crosspolymer | 0,15 | | | |
| PEG-150 Distearate | 0,50 | | | |
| Tapioca Starch + Aqua | 1,00 | | | |
| Ammonium Acryloyldimethyltaurate/VP Copolymer + Aqua | 0,50 | 0,65 | 0,65 | 0,65 |
| Methylpropanediol | | | 4,00 | 4,00 |
| Sodium Stearoyl Glutamate + Sodium Chloride | 0,30 | | | |
| | | | | |
| Tofa-Derivat T 105 e | 2,00 | | | |
| Tofa-Derivat T 71 e | | 1,00 | | |
| Tofa-Derivat T 74 e | | | 2,00 | |
| Tofa-Derivat T 113 e | | | | 2 |
| | | | | |
| | | | | |
| | | | | |
| **Wasser auf** | **100,00** | **100,00** | **100,00** | **100,00** |
| | | | | |

| | Beispiel 5 | Beispiel 6 | Beispiel 7 |
|---|---|---|---|
| Caprylic/Capric Triglyceride | | | 4,00 |
| Propylene Glycol + Aqua | 5,00 | | |
| Citric Acid | 0,00 | | |
| Cetyl Alcohol | | | 3,00 |
| Aqua + Trisodium EDTA | | 1,00 | 1,00 |
| Diisopropyl Adipate | | 10,00 | 10,00 |
| Phenoxyethanol + Aqua | | 0,50 | 0,80 |
| Hydrogenated Coco-Glycerides | | | 2,00 |
| Glycerin | 10,00 | 3,00 | 9,00 |
| Aqua + Sodium Hydroxide | | 0,06 | 0,01 |
| PEG-40 Hydrogenated Castor Oil | 5,00 | | |
| Butylene Glycol + Aqua | 10,00 | | 5,00 |
| Alcohol Denat. + Aqua | 40,00 | 10,00 | 5,00 |
| Xanthan Gum | | 0,30 | 0,30 |
| Acrylates/C10-30 Alkyl Acrylate Crosspolymer | | | 0,20 |
| Glyceryl Stearate Citrate | | | 2,00 |
| Acrylates/C10-30 Alkyl Acrylate Crosspolymer | | 0,20 | |
| Ammonium Acryloyldimethyltaurate/VP Copolymer + Aqua | 0,65 | | |
| Methylpropanediol | 4,00 | | |
| Tofa-Derivat T 105 e | 0,50 | | |
| | | 0,50 | |
| | | | 1 |
| | | | |
| | | | |
| **Wasser auf** | **100,00** | **100,00** | **100,00** |
| | | | |

## Patentansprüche

**1.** Verbindungen der allgemeinen Formel

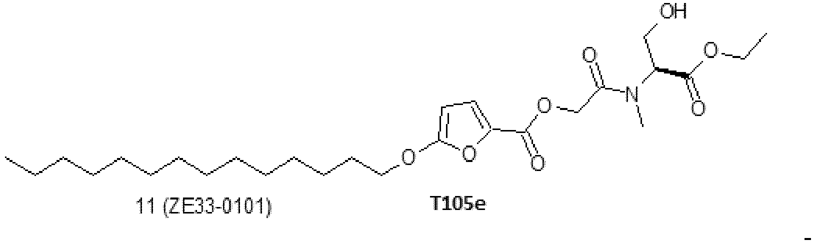

**3.** Medizinische oder kosmetische topische Zubereitungen, enthaltend eine oder mehrere Verbindungen der Formel gemäß Anspruch 1 in einer wirksamen Konzentration,

**4.** Medizinische oder kosmetische topische Zubereitungen, enthaltend eine oder mehrere Verbindungen der Formel gemäß Anspruch 1 in Konzentrationen von 0,0001 -40,00 Gew.-%, bevorzugt 0,005 - 20,00 Gew.-%, besonders bevorzugt 0,001 - 6,00 Gew.-%, jeweils bezogen auf das Gesamtgewicht der Zusammensetzung, vorliegen.

**5.** Kosmetische, nicht therapeutische Verwendung einer oder mehrerer Verbindungen der Formel gemäß Anspruch 1 zur Verringerung der Sebumproduktion in der menschlichen Haut,

**6.** Eine oder mehrere Verbindungen der Formel gemäß Anspruch 1 zur therapeutischen Behandlung von milden Formen der Akne und/oder der Seborrhö.
